(19) **Europäisches Patentamt / European Patent Office / Office européen des brevets**

(11) **EP 3 768 350 B1**

(12) **EUROPÄISCHE PATENTSCHRIFT**

(45) Veröffentlichungstag und Bekanntmachung des Hinweises auf die Patenterteilung:
**13.03.2024 Patentblatt 2024/11**

(21) Anmeldenummer: **19713017.2**

(22) Anmeldetag: **22.03.2019**

(51) Internationale Patentklassifikation (IPC):
**A61M 1/14** (2006.01)

(52) Gemeinsame Patentklassifikation (CPC):
**A61M 1/14;** A61M 2205/16; A61M 2205/17;
A61M 2205/52; A61M 2205/70

(86) Internationale Anmeldenummer:
**PCT/EP2019/057283**

(87) Internationale Veröffentlichungsnummer:
**WO 2019/180228 (26.09.2019 Gazette 2019/39)**

(54) **MEDIZINGERÄT**

MEDICAL DEVICE

APPAREIL MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.03.2018 DE 102018106906**

(43) Veröffentlichungstag der Anmeldung:
**27.01.2021 Patentblatt 2021/04**

(73) Patentinhaber: **Fresenius Medical Care Deutschland GmbH**
**61352 Bad Homburg (DE)**

(72) Erfinder:
• **BARDORZ, Christoph**
**97228 Rottendorf (DE)**

• **SIEBERT, Jochen**
**97688 Bad Kissingen (DE)**
• **KELLERMANN, Stefan**
**97711 Maßbach (DE)**

(74) Vertreter: **Nordmeyer, Philipp Werner df-mp Dörries Frank-Molnia & Pohlman Patentanwälte Rechtsanwälte PartG mbB Theatinerstraße 16 80333 München (DE)**

(56) Entgegenhaltungen:
| | |
|---|---|
| EP-A1- 1 698 361 | EP-B1- 1 698 361 |
| DE-A1- 4 131 247 | DE-B4- 4 131 247 |
| US-A1- 2013 190 674 | US-B1- 6 423 268 |

**Beschreibung**

Technisches Gebiet

Die vorliegende Erfindung betrifft ein Medizingerät zur Durchführung einer Behandlung an einem Patienten, bevorzugt eine Dialysevorrichtung, mittels welcher beispielsweise eine Hämodialyse, eine Hämofiltration oder eine Hämodiafiltration durchgeführt werden kann.

Stand der Technik

In Medizingeräten ist es von Bedeutung, die Integrität und die Sicherheit des Medizingeräts jederzeit während des Betriebs sicherzustellen. Hierzu werden Sicherheitsvorkehrungen innerhalb des Medizingeräts bereitgestellt, mittels welchen sich das Medizingerät während der Behandlung selbst überwacht. Auf diese Weise kann auch eine Ausfallsicherheit bereitgestellt werden und im Fehlerfalle können entsprechende Maßnahmen ergriffen werden, beispielsweise eine Fehlermeldung ausgegeben werden, ein Alarm ausgelöst werden und/oder die Behandlung gestoppt oder abgebrochen werden, um die Patientensicherheit jederzeit zu gewährleisten.

Beispielsweise sind in Medizingeräten, beispielsweise in Dialysevorrichtungen, redundante Steuermechanismen vorgesehen, wobei beispielsweise ein Hauptsteuermodul vorgesehen ist, welches die Steuerung der Dialysevorrichtung im Betrieb und bei der Behandlung übernimmt. Weiterhin ist ein unabhängig von dem Hauptsteuermodul betriebenes Schutzmodul vorgesehen, mittels welchem das Hauptsteuermodul überwacht wird. Das Schutzmodul kann beispielsweise in Form eines zweiten, im wesentlichen identisch zu dem Hauptsteuermodul aufgebauten Modul bereitgestellt werden, welches im Havariefall des Steuermoduls einspringen kann und das Medizingerät in einen sicheren Zustand bringen kann. Neben den genannten Hauptsteuermodulen und Schutzmodulen können in Medizingeräten auch zusätzliche Funktionsmodule vorgesehen sein, welche ebenfalls unabhängig von dem Hauptsteuermodul arbeiten und beispielsweise in Form eines Anzeigemoduls oder Monitormoduls vorgesehen sind. Einzelne funktionelle Komponenten und funktionale Untergruppen des Medizingeräts können ebenfalls mit unabhängig vom Hauptsteuermodul agierenden Funktionsmodulen betrieben werden. Die unterschiedlichen Funktionsmodule kommunizieren üblicher Weise über einen Bus und/oder definierte Schnittstellen miteinander.

Neben der Überwachung der Funktion des Medizingerätes im Betrieb vor der jeweiligen Behandlung des Patienten, während der Behandlung des Patienten und nach der Behandlung des Patienten, sowie bei routinemäßigen Test- und Wartungssequenzen ist als weitere Vorrichtung und/oder Schutzmechanismus des Medizingeräts ein Betriebsstundenzähler vorgesehen, mittels welchem die Gesamtbetriebszeit des Medizingeräts beziehungsweise die Betriebszeit einzelner Komponenten ermittelt werden kann. Entsprechend kann nach dem Ablauf einer bestimmten Betriebszeit eine Wartung bestimmter Komponenten oder des gesamten Medizingeräts durchgeführt werden, um die sichere Funktion des Medizingeräts weiterhin sicher zu stellen.

Nach dem Ablauf einer bestimmten Betriebszeit einzelner Komponenten kann auch ein Austausch der jeweiligen Komponente notwendig werden. Durch eine Dokumentation und Anzeige der abgelaufenen Betriebszeit kann entsprechend eine Wartung angestoßen werden oder ein Austausch von Komponenten initiiert werden. Der reinen Angabe beispielsweise eines Herstellungsdatums oder eines Verfallsdatums ist die Dokumentation der Betriebszeit überlegen, da die tatsächliche Verwendung des Medizingeräts auf diese Weise erfasst werden kann und sich aus der entsprechenden betriebsbedingten Abnutzung ein Wartungs- und Austauschbedarf ergeben kann. Dieser Wartungs- und Austauschbedarf ergibt sich hingegen nicht zwangsläufig aus einem starr vorgegebenen Datum, welches dazu führen kann, dass der Wartungsbedarf entweder zu frühzeitig abgerufen wird, so dass die Abnutzung der Komponenten nicht der tatsächlichen Nutzung des Medizingeräts entspricht und eine Wartung entsprechend unwirtschaftlich ist, oder der Wartungsbedarf zu spät abgerufen wird, so dass bei einer sehr starken Nutzung des Medizingerätes möglicherweise die Sicherheit des Medizingerätes gefährdet werden kann, da die Abnutzung der Komponenten schon zu weit fortgeschritten ist. Entsprechend ist aus wirtschaftlichen Gründen und aus Gründen der Betriebssicherheit eine Dokumentation der tatsächlichen Betriebsstunden bevorzugt.

Die Betriebsstunden können mittels eines mechanischen Zählers im Medizingerät gezählt werden, wobei ein mechanischer Betriebsstundenzähler jedoch mit Zusatzkosten verbunden ist und der mechanische Betriebsstundenzähler als mechanisches Bauteil einer Abnutzung und damit einer begrenzten Betriebssicherheit unterliegt.

Weiterhin bekannt sind Betriebsstundenzähler, welche in einem Hauptsteuermodul beziehungsweise auf der Hauptplatine des Medizingerätes elektronisch implementiert sind. Entsprechend verwendet ein Mikrocontroller den Prozessortakt, zählt auf dieser Grundlage die Betriebsstunden und speichert den ermittelten Betriebsstundenwert in einem nichtflüchtigen Speicher in dem Hauptsteuermodul ab. Bei einem Defekt des nichtflüchtigen Speichers oder des Hauptsteuermoduls oder bei einem Tausch des Hauptsteuermoduls oder der Hauptplatine wird jedoch der Betriebsstundenzähler entsprechend ungültig und muss von einem Servicetechniker aufwändig neu gesetzt werden.

In einer weiteren Implementierung, welche beispielsweise in den Dialysevorrichtungen 5008/6008 der Anmelderin vorliegt, verfügen einige der Funktionsmodule, also insbesondere ein Hauptsteuermodul, ein Schutzmodul und ein Monitormodul, jeweils über einen eigenen Betriebsstundenzähler, welche unabhängig voneinander die Betriebszeit des jeweiligen Funktionsmoduls auf Grundlage des Prozessortakts des Prozessors des jeweiligen Funktionsmoduls zählen. Nach dem Einschalten der Maschine werden die Betriebsstundenzähler der Funktionsmodule untereinander verglichen und dann miteinander synchronisiert, wenn die Abweichung zwischen den einzelnen Werten kleiner als eine Stunde ist. Ist die Abweichung hingegen größer, wird eine Fehlermeldung ausgegeben. Auch hier ergibt sich die Problematik, dass beim Austausch eines Moduls standardmäßig eine Fehlermeldung ausgegeben wird, da der Betriebsstundenzähler des neu eingesetzten Moduls zwangsläufig von den Werten der Betriebsstundenzähler der verbleibenden Module abweicht. Entsprechend muss auch hier von einem Servicetechniker nach einem Modultausch ein aufwendiger Abgleich der Betriebsstundenzähler durchgeführt werden.

<< bitte hier die neue Seite 3a einsetzen >>

## Darstellung der Erfindung

Ausgehend von dem bekannten Stand der Technik ist es eine Aufgabe der vorliegenden Erfindung, ein Medizingerät anzugeben, welches einen robusten Betriebsstundenzähler bereitstellt, welcher die Wartung des Medizingeräts vereinfacht.

Diese Aufgabe wird durch ein Medizingerät mit den Merkmalen des Anspruchs 1 gelöst. Vorteilhafte Weiterbildungen ergeben sich aus den Unteransprüchen, der vorliegenden Beschreibung sowie den Figuren.

Entsprechend wird ein Medizingerät, bevorzugt eine Dialysevorrichtung zur Durchführung einer Hämodialyse und/oder Hämofiltration und/oder Hämodiafiltration, umfassend ein erstes Funktionsmodul und ein zweites Funktionsmodul vorgeschlagen, wobei in dem ersten Funktionsmodul ein Betriebsstundenzähler zum Zählen der Betriebsstunden vorgesehen ist, und das erste Funktionsmodul dazu eingerichtet ist, einen vom Betriebsstundenzähler ermittelten Betriebsstundenwert in einem ersten Speicher des ersten Funktionsmoduls zu speichern, wobei im << die Seite 3a bitte zwischen den Zeilen 19 und 20 der Seite 3 einfügen >>

Aus der US 6,423,268 B1 ist ein Wärmetauschersystem für Blutkreisläufe bekannt, wobei für zwei Bedienungsoberflächen jeweils ein Zähler vorgesehen ist, welcher im angeschalteten Zustand eine aktuelle Verwendungszeit wiedergibt. Weiterhin ist aus der DE 41 31 247 A1 ein Betriebsstundenmodul mit einem Erweiterungsmodul mit weiteren Speicherfunktionen bekannt.

Die US 2013/0190674 A1 offenbart ein Spiegeln von gespeicherten Behandlungsdaten für eine Mehrzahl von Speichermodulen innerhalb desselben Funktionsmoduls. Ein individuelles Ermitteln der Betriebsstunden für einzelne Komponenten eines medizinischen Geräts, um einen Verschleißzustand zu bestimmen, ist weiterhin in der EP 1 698 361 A1 offenbart.

zweiten Funktionsmodul ein zweiter Speicher vorgesehen ist. Erfindungsgemäß ist das zweite Funktionsmodul dazu eingerichtet, den vom Betriebsstundenzähler des ersten Funktionsmoduls ermittelten Betriebsstundenwert im zweiten Speicher zu speichern.

Dadurch, dass in dem zweiten Funktionsmodul ein zweiter Speicher vorgesehen ist, der dazu eingerichtet ist, den vom Betriebsstundenzähler des ersten Funktionsmoduls ermittelten Betriebsstundenwert zu speichern, ist der von dem Betriebsstundenzähler ermittelte Betriebsstundenwert in zumindest zwei unterschiedlichen Speichern in zwei unterschiedlichen Funktionsmodulen hinterlegt. Eine solche redundante Speicherung des von einem Betriebsstundenzähler ermittelten Betriebsstundenwerts führt zu einem robusten Betriebsstundenzähler, so dass auch im Wartungsfall und/oder beim Austausch eines Funktionsmoduls der ursprünglich im Medizingerät ermittelte Betriebsstundenwert im Medizingerät verbleibt.

Weiterhin findet ein asynchrones Hochzählen der gleichen Betriebsstunden durch unterschiedliche Betriebsstundenzähler in unterschiedlichen Modulen nicht mehr statt und damit fällt die Notwendigkeit einer Synchronisierung im Regelbetrieb fort.

Weiterhin wird der von dem Betriebsstundenzähler des ersten Funktionsmoduls ermittelte Betriebsstundenwert entsprechend in mindestens einem weiteren Funktionsmodul direkt in einem Speicher gespeichert, sodass der durch den zentralen Betriebsstundenzähler ermittelte Zählerwert zwar redundant gespeichert ist, aber nicht von mehreren Modulen ermittelt wird. Damit ergibt sich, dass zumindest im Regelbetrieb ein Gleichlauf zwischen den in den unterschiedlichen Speichern der unterschiedlichen Funktionsmodule vorliegenden Betriebsstundenwerten vorliegt, da sie nur von einem einzigen Betriebsstundenzähler ermittelt wurden.

Beim Austausch eines Funktionsmoduls kann auf den in einem Speicher eines anderen Funktionsmoduls hinterlegten Betriebsstundenwert zurückgegriffen werden und dieser Betriebsstundenwert wird dann als der eigentliche Betriebsstundenwert zugrunde gelegt. Das Medizingerät nimmt dann entsprechend an, dass jeder in dem Medizingerät in einem Speicher hinterlegte Betriebsstundenwert den tatsächlich abgelaufenen Betriebsstunden des Medizingeräts entspricht. Entsprechend ist eine Plausibilitätsüberprüfung zunächst nicht notwendig.

Damit kann für die typischen Fälle, in welchen der Austausch eines Funktionsmoduls notwendig ist, vermieden werden, dass ein Techniker einen ungültig gewordenen Betriebsstundenzähler aufwändig neu setzen muss. Vielmehr kann auf den in einem anderen Funktionsmodul gespeicherten Betriebsstundenwert zurückgegriffen werden.

Mit anderen Worten wird es auf diese Weise ermöglicht, dass der von dem Betriebsstundenzähler ermittelte Betriebsstundenwert an unterschiedlichen Stellen beziehungsweise in unterschiedlichen Funktionsmodulen innerhalb des Medizingerätes hinterlegt wird, sodass durch das Bereitstellen dieser "Backups" beim Austausch eines oder mehrerer Funktionsmodule immer noch auf einen gültigen Betriebsstundenwert des Be-triebsstundenzählers zurückgegriffen werden kann, wel-cher dann bevorzugt automatisch von dem neu eingesetzten Funktionsmodul übernommen wird.

Erfindungsgemäß ist das erste Funktionsmodul ein Hauptsteuermodul, bevorzugt ein Betriebssystem und das zweite Funktionsmodul ist ein Monitorsystem, bevorzugt ein Schutzsystem. Erfindungsgemäß weisen das erste Funktionsmodul und das zweite Funktionsmodul jeweils einen eigenen Mikroprozessor auf. Mit anderen Worten sind die einzelnen Funktionsmodule gewissermaßen autark und besitzen einen eigenen Mikroprozessor und einen eigenen Speicher.

Bevorzugt sind mindestens zwei weitere Funktionsmodule neben dem ersten Funktionsmodul vorgesehen, welche jeweils einen eigenen Speicher aufweisen, wobei die weiteren Funktionsmodule jeweils dazu eingerichtet sind, den vom Betriebsstundenzähler des ersten Funktionsmoduls ermittelten Betriebsstundenwert in ihrem Speicher zu speichern, wobei besonders bevorzugt jedes Funktionsmodul einen eigenen Mikroprozessor aufweist.

Dadurch, dass mehrere Funktionsmodule vorgesehen sind, in denen der Betriebsstundenwert abgespeichert wird, kann eine noch höhere Redundanz bei der Speicherung des Betriebsstundenwerts erreicht werden und die Robustheit des Betriebsstundenzählers wird dadurch noch weiter erhöht. Beispielsweise kann auch beim Ausfall oder Austausch mehrerer Funktionsmodule immer noch auf den in einem Speicher eines weiteren Funktionsmoduls gespeicherten Betriebsstundenwert zurückgegriffen werden und auf diese Weise die tatsächlich abgelaufenen Betriebsstunden im Medizingerät vorgehalten werden und auch eine automatische Synchronisierung und/oder Wiederherstellung der ungültig gewordenen Betriebsstundenwerte vorgenommen werden.

Bevorzugt ist eine Kommunikationsschnittstelle zwischen dem ersten Funktionsmodul und dem zweiten Funktionsmodul vorgesehen, welche dazu eingerichtet ist, den von dem Betriebsstundenzähler des ersten Funktionsmoduls ermittelten Betriebsstundenwert an den zweiten Speicher zu übertragen, bevorzugt an jeden Speicher der weiteren Funktionsmodule. Mittels der Kommunikationsschnittstelle ist es weiterhin möglich, den in einem Speicher gespeicherten Betriebsstundenwert durch ein anderes Funktionsmodul auszulesen, um auf diese Weise in einem ersten Funktionsmodul zu ermitteln, ob der in einem zweiten Funktionsmodul gespeicherte Betriebsstundenwert von dem im ersten Funktionsmodul gespeicherten Wert abweicht.

In einer bevorzugten Weiterbildung ist eine Synchronisationsvorrichtung vorgesehen, die dazu eingerichtet ist, die in den Speichern der Funktionsmodule gespeicherten Betriebsstundenwerte automatisch miteinander zu synchronisieren und/oder die Betriebsstundenwerte automatisch wieder herzustellen. Besonders bevorzugt wird hier eine automatische Wiederherstellung der Betriebsstundenwerte durchgeführt, wenn es zu einer Abweichung der Werte nach einer Wartung oder dem Austausch einer Platine oder eines Funktionsmoduls kommt. Auf diese Weise kann ein Austausch von Modulen ohne weiteren Arbeitsaufwand bezüglich des Betriebsstundenzählers durchgeführt werden. Beispielsweise kann der Speicher auf der neu angeschlossenen Platine dann automatisch auf den dann noch in dem Me-dizingerät verbleibenden Speicher gespeicherten Be-triebsstundenwert gesetzt werden.

In der Synchronisationsvorrichtung wird bevorzugt eine Mehrheitsentscheidung getroffen, wenn mehr als zwei Speicher mit unterschiedlichen Betriebsstundenwerten vorliegen. Der sich auf dieser Grundlage als falsch erweisende Betriebsstundenwert wird automatisch wiederhergestellt.

Dabei kann aus Sicherheitsgründen bevorzugt auch der größte Betriebsstundenwert der als richtig erkannten Betriebsstundenwerte übernommen werden.

Der im ersten Funktionsmodul vorgesehene Speicher und/oder der in dem zweiten Funktionsmodul und/oder allen Funktionsmodulen vorgesehene Speicher ist bevorzugt als nichtflüchtiger Speicher ausgebildet.

Bevorzugt ist der erste Speicher des ersten Funktionsmoduls dazu eingerichtet, weitere Werte zu speichern, optional Konfigurationswerte und/oder Setupwerte und/oder Kalibrierwerte, wobei das zweite Funktionsmodul dazu eingerichtet ist, die im Speicher des ersten Funktionsmoduls gespeicherten weiteren Werte im zweiten Speicher zu speichern und optional die Synchronisationsvorrichtung dazu eingerichtet ist, die in den Speichern der Funktionsmodule gespeicherten weiteren Werte automatisch miteinander zu synchronisieren und/oder automatisch wieder herzustellen.

Damit kann auch für weitere Werte des Medizingeräts, beispielsweise Konfigurationswerte und/oder Setupwerte und/oder Kalibrierwerte eine redundante Speicherung dieser Werte im Medizingerät vorgenommen werden, so dass beispielsweise beim Austausch eines Funktionsmoduls eine automatische Synchronisierung dieser Werte vorgenommen werden kann. Damit kann erreicht werden, dass sich das Medizingerät nach dem Austausch eines Funktionsmoduls im Wesentlichen im gleichen Konfigurationszustand befindet, wie vor dem Austausch des Funktionsmoduls. Damit kann der Nutzers des Medizingeräts direkt nach dem Austausch des Funktionsmoduls mit der Nutzung des Medizingeräts fortfahren und muss keine aufwändige Neukonfiguration durchführen.

Die oben genannte Aufgabe wird weiterhin durch ein Verfahren zur Zählung der Betriebsstunden in einem Medizingerät mit den Merkmalen des Anspruchs 8 gelöst. Vorteilhafte Weiterbildungen ergeben sich aus der vorliegenden Beschreibung, den Figuren sowie den Unteransprüchen.

Entsprechend wird ein Verfahren zur Zählung der Betriebsstunden eines Medizingeräts, bevorzugt einer Dialysevorrichtung zur Durchführung einer Hämodialyse und/oder Hämofiltration und/oder Hämodiafiltration, vorgeschlagen, wobei das Medizingerät ein erstes Funktionsmodul und ein zweites Funktionsmodul umfasst und wobei in dem ersten Funktionsmodul die Betriebsstunden mit einem Betriebsstundenzähler ermittelt werden und der ermittelte Betriebsstundenwert in einem Speicher des ersten Funktionsmoduls gespeichert wird. Erfindungsgemäß wird der von dem Betriebsstundenzähler des ersten Funktionsmoduls ermittelte Betriebsstundenwert in einem von dem zweiten Funktionsmodul umfassten zweiten Speicher gespeichert.

Dadurch, dass der in dem Betriebsstundenzähler des ersten Funktionsmoduls ermittelte Betriebsstundenwert auch in einem Speicher mindestens eines zweiten Funktionsmoduls gespeichert wird, ist zum einen ein Gleichlauf der in den unterschiedlichen Funktionsmodulen redundant, quasi als Backups, gespeicherten Betriebsstundenwerten sichergestellt, und zum anderen kann beim Auftreten einer Abweichung eine einfache Synchronisierung und/oder ein einfaches Wiederherstellen des korrekten Betriebsstundenwerts aus einem oder auch mehreren der in den Speichern gespeicherten Betriebsstundenwerte erreicht werden. Damit kann ein einfacher Austausch von Funktionsmodulen durchgeführt werden, ohne dass die Werte des Betriebsstundenzählers verloren gehen und ohne zusätzlichen Aufwand eines Wartungstechnikers.

Vorteilhaft wird der von dem Betriebsstundenzähler des ersten Funktionsmoduls ermittelte Betriebsstundenwert in weiteren Speichern weiterer Funktionsmodule gespeichert, so dass noch eine höhere Redundanz erreicht wird.

Gemäß einer vorteilhaften Ausprägung des Verfahrens wird bei einer Abweichung der in den Speichern gespeicherten Betriebsstundenwerte eine automatische Synchronisierung und/oder Wiederherstellung der Betriebsstundenwerte durchgeführt. Damit kann ohne weiteren Arbeitsaufwand des Servicetechnikers eine Wiederherstellung des von dem Betriebsstundenzähler ermittelten Betriebsstundenwerts beispielsweise beim Austausch eines Funktionsmoduls erreicht werden, wodurch ein besonders robustes und effizientes Verfahren zum Zählen der Betriebsstunden eines Medizingeräts bereitgestellt werden kann.

Weiterhin kann die automatische Synchronisierung und/oder Wiederherstellung der Betriebsstundenwerte nach einer Benutzereingabe und/oder nach einer Wartung und/oder nach dem Einschalten des Medizingeräts und/oder turnusgemäß durchgeführt werden.

Eine automatische Synchronisierung und/oder Wiederherstellung der Betriebsstundenwerte kann auch bei einer vorgegebenen Mindestabweichung der in den Speichern gespeicherten Betriebsstundenwerte durchgeführt werden, um im Medizingerät einen Gleichlauf der gespeicherten Betriebsstundenwerte zu erreichen.

Vorteilhaft kann der am häufigsten in den Speichern vorliegende Betriebsstundenwert als Grundlage für die Synchronisierung und/oder Wiederherstellung der Betriebsstundenwerte verwendet werden. Es wird quasi eine Mehrheitsentscheidung getroffen und auf dieser Grundlage dann entschieden, welcher der hinterlegten Betriebsstundenwerte schließlich als maßgebend angesehen wird.

Bevorzugt werden im ersten Speicher des ersten Funktionsmoduls weitere Werte gespeichert, optional Konfigurationswerte und/oder Setupwerte und/oder Kalibrierwerte und im zweiten Funktionsmodul werden die im Speicher des ersten Funktionsmoduls gespeicherten weiteren Werte entsprechend im zweiten Speicher gespeichert und optional werden die in den Speichern der Funktionsmodule gespeicherten weiteren Werte automatisch miteinander synchronisiert und/oder automatisch wieder hergestellt.

Kurze Beschreibung der Figuren

Bevorzugte weitere Ausführungsformen der Erfindung werden durch die nachfolgende Beschreibung der Figuren näher erläutert. Dabei zeigen:

Figur 1     eine schematische Darstellung eines Medizingeräts mit mehreren Funktionsmodulen; und

Figur 2     eine schematische Darstellung eines Dialysegeräts.

Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

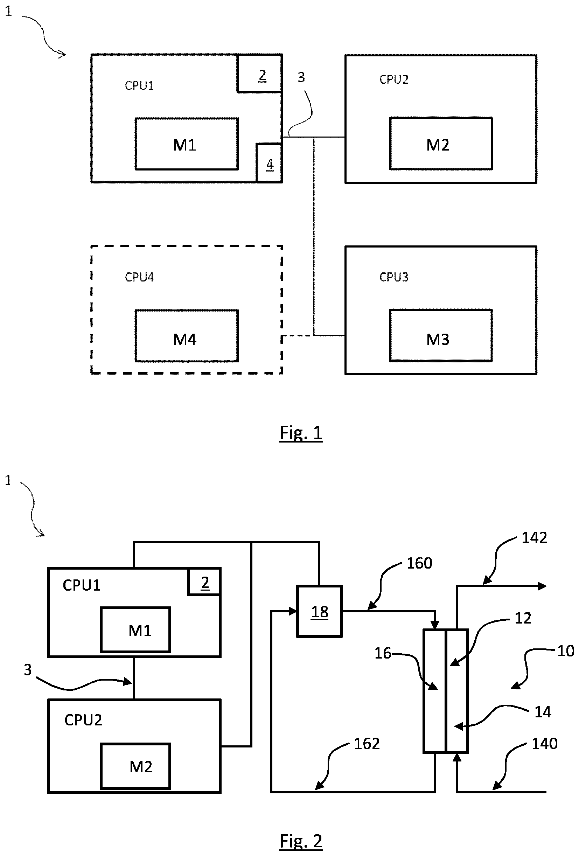

Figur 1 zeigt schematisch den Aufbau eines Medizingeräts 1, beispielsweise in Form einer Dialysevorrichtung zur Durchführung einer Hämodialyse und/oder Hämofiltration und/oder Hämodiafiltration.

In dem Medizingerät 1 ist ein erstes Funktionsmodul CPU1 vorgesehen, das in Form eines Hauptsteuermoduls vorgesehen ist, welches die Steuerung des Medizingeräts 1 übernimmt. Mit dem ersten Funktionsmodul CPU1 wird entsprechend beispielsweise die Behandlung des Patienten mit dem Medizingerät gesteuert.

Das erste Funktionsmodul CPU1 weist einen Mikroprozessor und einen Speicher M1 auf. Der Speicher M1 ist bevorzugt in Form eines nichtflüchtigen Speichers aufgebaut, um eine Speicherung unabhängig von der Stromversorgung des Funktionsmoduls CPU1 zu gewährleisten.

In dem Medizingerät ist weiterhin ein zweites Funktionsmodul CPU2 vorgesehen, welches ebenfalls einen Mikroprozessor und einen zweiten nichtflüchtigen Speicher M2 aufweist. Das erste Funktionsmodul CPU1 ist als Hauptsteuermodul, bevorzugt als Betriebssystem und das zweite Funktionsmodul CPU2 als Monitorsystem, bevorzugt als Schutzsystem aufgebaut.

Die beiden Funktionsmodule CPU1 und CPU2 können redundant zueinander aufgebaut sein, so dass das Schutzsystem CPU2 das Hauptsteuermodul CPU1 überwachen kann. Im Falle eines Ausfalles des Hauptsteuersystems CPU1 kann das Schutzsystem CPU2 einspringen und das Medizingerät 1 entsprechend in einen sicheren Zustand überführen und/oder einen Alarm ausgeben und/oder eine Fehlermeldung ausgeben.

Weiterhin ist in dem Medizingerät 1 ein weiteres Funktionsmodul CPU3 vorgesehen, welches beispielsweise als Monitorsystem ausgebildet sein kann. Es können weitere - in der Figur 1 gestrichelt angedeutete - Funktionsmodule vorgesehen sein, beispielsweise in Form des Funktionsmoduls CPU4, welche weitere Funktionen innerhalb des Medizingerätes 1 übernehmen. Die Funktionsmodule CPU1, CPU2, CPU3, CPU4 weisen jeweils einen eigenen, bevorzugt nichtflüchtigen, Speicher M1, M2, M3, M4 auf.

In dem ersten Funktionsmodul CPU1 ist weiterhin ein Betriebsstundenzähler 2 vorgesehen, mittels welchem die Betriebsstunden des Medizingerätes 1 gezählt und als Betriebsstundenwert ausgegeben werden können. Der Betriebsstundenzähler 2 verwendet dazu beispielsweise den Prozessortakt des Mikroprozessors des ersten Funktionsmoduls CPU1 und zählt entsprechend nach dem Einschalten des Medizingerätes 1 den Betriebsstundenwert hoch. Alternativ kann auch die Behandlungszeit gezählt werden oder ein anderer Zeitwert, welcher in dem jeweiligen Medizingerät 1 als Betriebszeit gemessen werden soll.

Der vom Betriebsstundenzähler 2 in dem ersten Funktionsmodul CPU1 ermittelte Betriebsstundenwert wird in dem nichtflüchtigen Speicher M1 des ersten Funktionsmoduls CPU1 gespeichert.

Die Speicherung kann beispielsweise jede Minute oder in anderen kürzeren oder längeren Zeitintervallen durchgeführt werden. Die Speicherung kann auch ausschließlich oder zusätzlich beim Ausschalten des Geräts und/oder zu Beginn und/oder nach Abschluss einer Behandlung durchgeführt werden.

Der von dem in dem ersten Funktionsmodul CPU1 vorgesehenen Betriebsstundenzähler 2 ermittelte Betriebsstundenwert wird weiterhin über eine Kommunikationsschnittstelle 3 beispielsweise an das zweite Funktionsmodul CPU2, an das dritte Funktionsmodul CPU3 und je nach Implementierung - auch an das vierte Funktionsmodul CPU4 und/oder an weitere Funktionsmodule kommuniziert und jeweils ebenfalls in deren Speichern M2 und M3 und - je nach Implementierung auch im optional vorgesehenen Speicher M4 und/oder den Speichern optional vorgesehener anderer Funktionsmodule abgelegt. Entsprechend findet im Regelbetrieb und je nach Implementierung ein Gleichlauf der Betriebsstundenwerte in allen Speichern M1, M2, M3 und optional M4 der jeweiligen Funktionsmodule CPU1, CPU2, CPU3 und optional CPU4 statt.

Muss ein Funktionsmodul ausgetauscht werden, beispielsweise das zweite Funktionsmodul CPU2, so wird beim Einsetzen des Austauschfunktionsmoduls CPU 2' entsprechend eine Initialisierung durchgeführt und ein Abgleich der in den Speichern M1, M2', M3 und optional M4 hinterlegten Betriebsstundenwerte durchgeführt.

In dem Fall des Austauschs des Funktionsmoduls wird festgestellt, dass in dem ausgetauschten Funktionsmodul CPU2' der in dessen Speicher M2' hinterlegte Betriebsstundenwert nicht mit den Betriebsstundenwerten, die in den anderen Speichern M1, M3 und optional M4 hinterlegt sind, übereinstimmt. Entsprechend wird mittels einer Synchronisationsvorrichtung 4, welche beispielsweise im ersten Funktionsmodul CPU1 vorgesehen sein kann, der im Speicher M2' des ausgetauschten Funktionsmoduls CPU2' vorliegende Betriebsstundenwert auf Grundlage der in den Speichern M1, M3 und optional M4 der anderen Funktionsmodule CPU1, CPU3 und optional CPU4 hinterlegten Betriebsstundenwerte wiederhergestellt.

Entsprechend findet ein automatisches Wiederherstellen und/oder Synchronisieren der Betriebsstundenwerte in allen Speichern M1, M2', M3 und optional M4 der Funktionsmodule CPU1, CPU2', CPU3 und optional CPU4 statt, sodass wiederum ein Gleichlauf der Betriebsstundenwerte vorliegt. Damit muss ein Abgleich oder ein Rücksetzen der Speicher durch einen Servicetechniker nicht durchgeführt werden, sondern es findet ein automatischer Abgleich statt.

Liegt eine Abweichung der einzelnen Betriebsstundenwerte in einzelnen Speichern M1, M2, M3 und optional M4 vor, so wird beispielsweise ab dem Vorliegen einer Mindestabweichung beispielsweise eine Mehrheitsentscheidung durch die Synchronisationsvorrichtung 4 getroffen, dahingehend, dass demjenigen Betriebsstundenwert der Vorrang gegeben wird, welcher in der größeren Anzahl in den Speichern M1, M2, M3 und optional M4 vorliegt. Es versteht sich, dass eine Mehrheitsentscheidung bereits bei drei Speichern M1, M2, M3 getroffen werden kann und der zusätzliche optionale Speicher M4 oder die Speicher weiterer optionaler Funktionsmodule hierfür nicht notwendig sind, bei deren Vorliegen aber in die Entscheidung mit einbezogen werden können, aber nicht müssen.

Im Falle eines Austausches eines Funktionsmoduls CPU2' ist dies entsprechend aufgrund des Gleichlaufs der Betriebsstundenwerte in allen Funktionsmodulen CPU1, CPU3 und optional CPU4 der Fall, außer in dem neu eingesetzten Funktionsmodul CPU2'. Damit kann entsprechend ein automatisches Wiederherstellen der Betriebsstundenwerte in allen Speichern M1, M2, M3 und optional M4 erreicht werden.

Auf diese Weise wird entsprechend auch ein Back-up des Betriebsstundenwertes, welcher von dem Betriebsstundenzähler 2 im ersten Funktionsmodul CPU1 ermittelt wird, in den Speichern M2, M3 und optional M4 der anderen Funktionsmodule CPU2, CPU3 und optional CPU4 erreicht. Damit ist ein einfacher Austausch von Funktionsmodulen möglich, ohne dass die Information über die abgelaufene Betriebszeit des Medizingerätes 1 verloren geht.

In einer beispielhaften Ausbildung wird für eine Wiederherstellung des Betriebsstundenwerts bei einer Abweichung kleiner oder gleich von 5 Stunden der einzelnen Betriebsstundenwerte in den Speichern M2, M3, M4 von dem ersten Speicher M1 in dem ersten Funktionsmodul CPU1 der im ersten Speicher M1 hinterlegte Betriebsstundenwert als gültig angenommen. Die Betriebsstundenwerte in den Speichern M2, M3 und optional M4 werden gegebenenfalls auf den aus dem ersten Speicher M1 entnommenen Betriebsstundenwert gesetzt.

Bei einer Abweichung größer 5 Stunden wird eine Mehrheitsentscheidung dahingehend getroffen, welcher der Speicher M1, M2, M3 und optional M4 fehlerhaft ist und der falsche Betriebsstundenwert dann automatisch korrigiert. Dabei wird aus Sicherheitsgründen der größte Betriebsstundenwert aller als richtig erkannten Betriebsstundenwerte übernommen.

Die Gültigkeit wird folgendermaßen ermittelt: Wenn |M1 - M2| ≤ 5h gilt, dann werden der Betriebsstundenwerte in dem Speicher M1 der CPU1 und der Betriebsstundenwert in dem Speicher M2 der CPU2 als gültig erkannt. Allgemein müssen also folgende Vergleiche der in den jeweils genannten Speichern gespeicherten Betriebsstundenwerte durchgeführt werden

$$|M1 - M2| \leq MAX\_DEV$$

$$|M1 - M3| \leq MAX\_DEV$$

$$|M2 - M3| \leq MAX\_DEV$$

Ist keine Korrektur möglich (beispielsweise, weil die Abweichung zwischen allen Betriebsstundenwerten größer als 5 Stunden ist), wird eine Fehlermeldung ausgegeben.

Neben den Betriebsstundenwerten können in den Speichern M1, M2, M3 und optional M4 der Funktionsmodule CPU1, CPU2, CPU3 und optional CPU4 auch weitere Werte des Medizingeräts 1 redundant beziehungsweise mit einem Backup gespeichert werden. Beispielsweise können Konfigurationswerte und/oder Setupwerte und/oder Kalibrierwerte aus dem ersten Funktionsmodul CPU1 in den Speichern M1, M2, M3 und optional M4 gespeichert werden und mittels der Synchronisationsvorrichtung 4 automatisch synchron gehalten werden, so dass im Falle eines Austausches eines Funktionsmoduls CPU1, CPU2, CPU3 und optional CPU4 und insbesondere des ersten Funktionsmoduls wieder automatisch auf diese Konfigurationswerte und/oder Setupwerte und/oder Kalibrierwerte zurückgegriffen werden kann.

In Figur 2 ist das Medizingerät 1 in einer weiteren Ausführungsform als Dialysevorrichtung ausgebildet. Entsprechend weist die Vorrichtung einen Dialysator 10 auf, welcher einen Blutraum 14 und einen Dialysatraum 16 umfasst, wobei beide Räume durch eine Membran 12 voneinander getrennt sind. Dialysatoren der hier angesprochenen Art sind allgemein bekannt. Sie weisen ein Gehäuse auf, in dem beispielsweise mehrere zu einem Hohlfaserbündel zusammengefasste zylinderförmige Hohlfasermembranen vorgesehen sind. Durch das Innere der Hohlfasermembranen fließt Blut, während im Raum zwischen den Hohlfasermembranen und dem Filtergehäuse ein Dialysat fließt. Selbstverständlich können auch anders ausgebildete Dialysatoren vorgesehen sein.

Dementsprechend wird vorliegend unter Blutraum 14 die Summe der Bereiche verstanden, in denen Blut im Dialysator 10 fließt und unter Dialysatraum 16 die Summe der Bereiche, in denen ein Dialysat fließt. Die Wand der Membran des Dialysators 10 ist semipermeabel ausgebildet, sodass ein Stoffaustausch zwischen dem Blut und dem Dialysat erfolgen kann. Bei der Hämodialyse erfolgt dieser durch Diffusion, solange bis sich ein Konzentrationsgleichgewicht in dem Blutraum 14 und dem Dialysatraum 16 einstellt. Je nach der Porengröße der Membranen werden größere oder kleinere Moleküle in den Dialysatraum 9 durchgelassen.

Im Betrieb der Dialysevorrichtung 1 ist ein Patient an diese angeschlossen. Über eine Blutzuführung 140 fließt das Blut des Patienten in den Blutraum 14 des Dialysators 10 und von dort über die Rückführung 142 zurück zum Patienten. In dem Dialysatraum 16 des Dialysators 10 fließt das Dialysat vorzugsweise im Gegenstrom zu dem Blutfluss, wie in Figur 2 durch die Pfeile angedeutet ist. Während der Dialyse findet der oben beschriebene Stoffaustausch zwischen dem Blut und dem Dialysat statt, wobei letzteres anschließend den Dialysator 3 über die Leitung 162 verlässt. Das Dialysat enthält dabei die Substanzen, die während der Dialyse aus dem Blutkreislauf entfernt werden.

Das Dialysat wird durch eine Dialysatvorrichtung 18 bereitgestellt. Die Dialysatvorrichtung 18 ermöglicht es, dass das Dialysat über eine Dialysatleitung 160 in den Dialysatraum 16 fließt und über die Dialysatleitung 162 den Dialysatraum 16 verlässt und wieder zur Dialysatvorrichtung 18 zurückfließt. Das verwendete Dialysat kann dabei in der Dialysatvorrichtung 18 entweder vom frischen Dialysat getrennt und entsorgt oder zumindest teilweise durch entsprechende Aufreinigung wieder verwendet werden. Es kann ebenfalls vorgesehen sein, dass das Dialysat bereits stromaufwärts der Dialysatvorrichtung 18 entsorgt wird.

Zum Fördern des Dialysats in dem entsprechend gebildeten Dialysatkreislauf, ist eine Dialysatpumpe (nicht gezeigt) vorgesehen, welche beispielsweise stromabwärts oder stromaufwärts des Dialysators 10 oder auch in der Dialysatvorrichtung 18 angeordnet sein kann.

Entsprechend ist ebenfalls eine Blutpumpe zum Fördern des Bluts im Blutkreislauf des Dialysators 10 vorgesehen (nicht gezeigt). Es können zudem weitere Dialysekomponenten, wie beispielsweise Ventile, Klemmen, Tropfkammern, Druckmesser oder Antikoagulanz-Pumpen vorgesehen sein, welche jedoch nicht im Detail in der Ausführungsform gemäß Figur 2 gezeigt sind.

Ebenfalls sind in der Dialysevorrichtung 1 zwei Funktionsmodule CPU1, CPU2 angeordnet, welche den Funktionsmodulen gemäß Figur 1 im Wesentlichen entsprechen.

Die Funktionsmodule CPU1, CPU2 sind miteinander über eine Kommunikationsstelle 3 verbunden, sodass der Betriebsstundenwert, welcher von dem im ersten Funktionsmodul CPU1 vorgesehenen Betriebsstundenzähler 2 gezählt wird, in dem nichtflüchtigen Speicher M1 des ersten Funktionsmoduls CPU1 gespeichert und weiterhin über eine Kommunikationsschnittstelle 3 an das zweite Funktionsmodul CPU2 übermittelt und in deren entsprechendem Speicher M2 gespeichert wird. Entsprechend findet im Regelbetrieb und je nach Implementierung ein Gleichlauf der Betriebsstundenwerte in allen Speichern M1 und M2 der jeweiligen Funktionsmodule CPU1, CPU2 statt.

Obwohl nicht im Detail gezeigt, können optional ebenfalls weitere Funktionsmodule und/oder eine Synchronisationsvorrichtung vorgesehen sein, wie beispielsweise für die Ausführungsform gemäß Figur 1 beschrieben.

Das erste Funktionsmodul CPU1 und das zweite Funktionsmodul sind in der Figur 2 als separate Funktionsmodule dargestellt, welche jeweils mit der Dialysatvorrichtung 18 kommunikativ verbunden sind. Erfindungsgemäß ist das erste Funktionsmodul CPU1 als Hauptsteuermodul, bevorzugt als Betriebssystem vorgesehen, welches die Steuerung des Medizingeräts 1 übernimmt. Mit dem ersten Funktionsmodul CPU1 wird entsprechend beispielsweise die Behandlung des Patienten mit dem Medizingerät gesteuert. Das zweite Funktionsmodul CPU2 erfüllt dabei beispielsweise die Funktion eines Schutzsystems, welches das Hauptsteuermodul CPU1 überwachen und im Falle eines Ausfalls des Hauptsteuersystems CPU1 das Medizingerät 1 entsprechend in einen sicheren Zustand überführen kann. Das zweite Funktionsmodul CPU2 kann dabei redundant zum Hauptsteuermodul ausgebildet sein.Obwohl die Funktionsmodule CPU1, CPU2, wie in Figur 2 dargestellt, mit der Dialysatvorrichtung 18 verbunden sind, können diese, alternativ oder zusätzlich, ebenfalls mit anderen Komponenten der Dialysevorrichtung 1 verbunden sein, um entsprechend anderer Funktionen der Dialysebehandlung zu steuern/regeln. Beispielsweise können die Funktionsmodule mit Pumpen und/oder Ventilen, welche das Blut bzw. das Dialysat im entsprechenden Kreislauf fördern, verbunden sein. Weiterhin können die Funktionsmodule in einem separaten Gehäuse untergebracht oder ebenfalls in entsprechenden Komponenten integriert sein, beispielsweise als integrierte Steuervorrichtung, wobei der Betriebsstundenwert in unterschiedlichen Speichermedien gespeichert ist.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

Bezugszeichenliste

| | |
|---|---|
| 1 | Medizingerät |
| 10 | Dialysator |
| 12 | Membran |
| 14 | Blutraum |
| 140 | Blutzuführung |
| 142 | Rückführung |
| 16 | Dialysatraum |
| 160 | Dialysatleitung |
| 162 | Dialysatleitung |
| 18 | Dialysatvorrichtung |
| 2 | Betriebsstundenzähler |
| 3 | Kommunikationsschnittstelle |
| 4 | Synchronisationsvorrichtung |
| CPU1 | erstes Funktionsmodul |
| CPU2 | zweites Funktionsmodul |
| CPU3 | drittes Funktionsmodul |
| CPU4 | viertes, optionales Funktionsmodul |
| M1 | erster Speicher |
| M2 | zweiter Speicher |
| M3 | dritter Speicher |
| M4 | vierter, optionaler Speicher |

**Patentansprüche**

1. Medizingerät (1), bevorzugt Dialysevorrichtung zur Durchführung einer Hämodialyse und/oder Hämofiltration und/oder Hämodiafiltration, umfassend ein erstes Funktionsmodul (CPU1) und ein zweites Funktionsmodul (CPU2), wobei in dem ersten Funktionsmodul (CPU1) ein Betriebsstundenzähler (2) zum Zählen der Betriebsstunden vorgesehen ist, und das erste Funktionsmodul (CPU1) dazu eingerichtet ist, einen vom Betriebsstundenzähler (2) ermittelten Betriebsstundenwert in einem ersten Speicher (M1) des ersten Funktionsmoduls (CPU1) zu speichern, wobei im zweiten Funktionsmodul (CPU2) ein zweiter Speicher (M2) vorgesehen ist, **dadurch gekennzeichnet, dass**

das zweite Funktionsmodul (CPU2) dazu eingerichtet ist, den vom Betriebsstundenzähler (2) des ersten Funktionsmoduls (CPU1) ermittelten Betriebsstundenwert im zweiten Speicher (M2) zu speichern, wobei das erste Funktionsmodul (CPU1) ein Hauptsteuermodul und das zweite Funktionsmodul (CPU2) ein Monitorsystem ist, und das erste Funktionsmodul (CPU1) und das zweite Funktionsmodul (CPU2) jeweils einen eigenen Mikroprozessor aufweisen.

2. Medizingerät (1) gemäß Anspruch 1, **dadurch gekennzeichnet, dass** das erste Funktionsmodul (CPU1) ein Betriebssystem ist und/oder das zweite Funktionsmodul (CPU2) ein Schutzsystem ist.

3. Medizingerät (1) gemäß Anspruch 1 oder 2, **dadurch gekennzeichnet, dass** mindestens zwei weitere Funktionsmodule (CPU2, CPU3, CPU4) zusätzlich zu dem ersten Funktionsmodul (CPU1) vorgesehen sind, welche jeweils einen eigenen Speicher (M2, M3, M4) aufweisen, wobei die weiteren Funktionsmodule (CPU2, CPU3, CPU4) jeweils dazu eingerichtet sind, den vom Betriebsstundenzähler (2) des ersten Funktionsmoduls (CPU1) ermittelten Betriebsstundenwert in ihrem Speicher (M2, M3, M4) zu speichern und wobei bevorzugt jedes Funktionsmodul (CPU1, CPU2, CPU3, CPU4) einen eigenen Mikroprozessor aufweist.

4. Medizingerät (1) gemäß einem der vorstehenden Ansprüche, **dadurch gekennzeichnet, dass** eine Kommunikationsschnittstelle (3) zwischen dem ersten Funktionsmodul (CPU1) und dem zweiten Funktionsmodul (CPU2) vorgesehen ist, welche dazu eingerichtet ist, den von dem Betriebsstundenzähler (2) des ersten Funktionsmoduls (CPU1) ermittelten Betriebsstundenwert an den zweiten Speicher (M2) zu übertragen, bevorzugt an jeden Speicher (M2, M3, M4) der weiteren Funktionsmodule (CPU2, CPU3, CPU4).

5. Medizingerät (1) gemäß einem der vorstehenden Ansprüche, **dadurch gekennzeichnet, dass** eine Synchronisationsvorrichtung (4) vorgesehen ist, die dazu eingerichtet ist, die in den Speichern (M1, M2, M3, M4) der Funktionsmodule (CPU1, CPU2, CPU3, CPU4) gespeicherten Betriebsstundenwerte automatisch miteinander zu synchronisieren und/oder die Betriebsstundenwerte automatisch wiederherzustellen.

6. Medizingerät (1) gemäß einem der vorstehenden Ansprüche, **dadurch gekennzeichnet, dass** der im ersten Funktionsmodul (CPU1) vorgesehen Speicher (M1) und/oder der in dem zweiten Funktionsmodul (CPU2) und/oder allen Funktionsmodulen (CPU2, CPU3, CPU4) vorgesehene Speicher (M2, M3, M4) ein nichtflüchtiger Speicher ist.

7. Medizingerät (1) gemäß einem der vorstehenden Ansprüche, **dadurch gekennzeichnet, dass** der erste Speicher (M1) des ersten Funktionsmoduls (CPU1) dazu eingerichtet ist, weitere Werte zu speichern, bevorzugt Konfigurationswerte und/oder Setupwerte und/oder Kalibrierwerte, wobei das zweite Funktionsmodul (CPU2) dazu eingerichtet ist, die im Speicher (M1) des ersten Funktionsmoduls (CPU1) gespeicherten weiteren Werte im zweiten Speicher (M2) zu speichern und die Synchronisationsvorrichtung (4) bevorzugt dazu eingerichtet ist, die in den Speichern (M1, M2, M3, M4) der Funktionsmodule (CPU1, CPU2, CPU3, CPU4) gespeicherten weiteren Werte automatisch miteinander zu synchronisieren und/oder automatisch wiederherzustellen.

8. Verfahren zur Zählung der Betriebsstunden eines Medizingeräts, bevorzugt einer Dialysevorrichtung zur Durchführung einer Hämodialyse und/oder Hämofiltration und/oder Hämodiafiltration, welches ein erstes Funktionsmodul (CPU1) und ein zweites Funktionsmodul (CPU2) umfasst, wobei in dem ersten Funktionsmodul (CPU1) die Betriebsstunden mit einem Betriebsstundenzähler (2) ermittelt werden und der ermittelte Betriebsstundenwert in einem Speicher (M1) des ersten Funktionsmoduls (CPU1) gespeichert wird,
**dadurch gekennzeichnet, dass**
der von dem Betriebsstundenzähler (2) des ersten Funktionsmoduls (CPU1) ermittelte Betriebsstundenwert in einem von dem zweiten Funktionsmodul (CPU2) umfassten zweiten Speicher (M2) gespeichert wird, wobei das erste Funktionsmodul (CPU1) ein Hauptsteuermodul und das zweite Funktionsmodul (CPU2) ein Monitorsystem ist.

9. Verfahren gemäß Anspruch 8, **dadurch gekennzeichnet, dass** der von dem Betriebsstundenzähler (2) des ersten Funktionsmoduls (CPU1) ermittelte Betriebsstundenwert in weiteren Speichern (M2, M3, M4) weiterer Funktionsmodule (CPU2, CPU3, CPU4) gespeichert wird.

10. Verfahren gemäß Anspruch 8 oder 9, **dadurch gekennzeichnet, dass** bei einer Abweichung der in den Speichern (M1, M2, M3, M4) gespeicherten Betriebsstundenwerte eine automatische Synchronisierung und/oder Wiederherstellung der Betriebsstundenwerte durchgeführt wird.

11. Verfahren gemäß Anspruch 10, **dadurch gekennzeichnet, dass** die automatische Synchronisierung und/oder Wiederherstellung der Betriebsstundenwerte nach einer Benutzereingabe und/oder nach einer Wartung und/oder nach dem Einschalten des Medizingeräts (1) und/oder turnusgemäß durchgeführt wird.

**12.** Verfahren gemäß Anspruch 10 oder 11, **dadurch gekennzeichnet, dass** die automatische Synchronisierung und/oder Wiederherstellung der Betriebsstundenwerte bei einer vorgegebenen Mindestabweichung der in den Speichern (M1, M2, M3, M4) gespeicherten Betriebsstundenwerte durchgeführt wird.

**13.** Verfahren gemäß einem der Ansprüche 10 bis 12, **dadurch gekennzeichnet, dass** der am häufigsten in den Speichern (M1, M2, M3, M4) vorliegende Betriebsstundenwert als Grundlage für die Synchronisierung und/oder Wiederherstellung der Betriebsstundenwerte verwendet wird.

**14.** Verfahren gemäß einem der Ansprüche 8 bis 13, **dadurch gekennzeichnet, dass** im ersten Speicher (M1) des ersten Funktionsmoduls (CPU1) weitere Werte gespeichert werden, bevorzugt Konfigurationswerte und/oder Setupwerte und/oder Kalibrierwerte und im zweiten Funktionsmodul (CPU2) die im Speicher (M1) des ersten Funktionsmoduls (CPU1) gespeicherten weiteren Werte im zweiten Speicher (M2) gespeichert werden und die in den Speichern (M1, M2, M3, M4) der Funktionsmodule (CPU1, CPU2, CPU3, CPU4) gespeicherten weiteren Werte bevorzugt automatisch miteinander synchronisiert werden und/oder automatisch wiederhergestellt werden.

**Claims**

**1.** Medical device (1), preferably a dialysis device for performing a haemodialysis and/or haemofiltration and/or haemodiafiltration, comprising a first functional module (CPU1) and a second functional module (CPU2), wherein an operating hours counter (2) for counting the operating hours is provided in the first functional module (CPU1), and the first functional module (CPU1) is configured to store an operating hours value determined by the operating hours counter (2) in a first storage (M1) of the first functional module (CPU1), wherein a second storage (M2) is provided in the second functional module (CPU2), **characterised in that** the second functional module (CPU2) is configured to store the operating hours value determined by the operating hours counter (2) of the first functional module (CPU1) in the second storage (M2), wherein the first functional module (CPU1) is a main control module and the second functional module (CPU2) is a monitoring system, and the first functional module (CPU1) and the second functional model (CPU2) each have an individual microprocessor.

**2.** Medical device (1) according to claim 1, **characterised in that** the first functional module (CPU1) is an operating system and/or the second functional module (CPU2) is a protection system.

**3.** Medical device (1) according to claim 1 or 2, **characterised in that** at least two further functional modules (CPU2, CPU3, CPU4) are provided in addition to the first functional module (CPU1), which each have an individual storage (M2, M3, M4), wherein the further functional modules (CPU2, CPU3, CPU4) are each configured to store the operating hours value determined by the operating hours counter (2) of the first functional module (CPU1) in the respective storage (M2, M3, M4), and wherein each functional module (CPU1, CPU2, CPU3, CPU4) preferably has an individual microprocessor.

**4.** Medical device (1) according to any one of the preceding claims, **characterised in that** a communication interface (3) is provided between the first functional module (CPU1) and the second functional module (CPU2), and is configured to transmit the operating hours value determined by the operating hours counter (2) of the first functional module (CPU1) to the second storage (M2), preferably to each storage (M2, M3, M4) of the further functional modules (CPU2, CPU3, CPU4).

**5.** Medical device (1) according to any one of the preceding claims, **characterised in that** a synchronisation device (4) is provided which is configured to automatically synchronise the operating hours values stored in the storages (M1, M2, M3, M4) of the functional modules (CPU1, CPU2, CPU3, CPU4) with each other and/or to automatically restore the operating hours values.

**6.** Medical device (1) according to any one of the preceding claims, **characterised in that** the storage (M1) provided in the first functional module (CPU1) and/or the storage (M2, M3, M4) provided in the second functional module (CPU2) and/or all functional modules (CPU2, CPU3, CPU4) is a non-volatile storage.

**7.** Medical device (1) according to any one of the preceding claims, **characterised in that** the first storage (M1) of the functional module (CPU1) is configured to store further values, preferably configuration values and/or setup values and/or calibration values, wherein the second functional module (CPU2) is configured to store the further values stored in the storage (M1) of the first functional module (CPU1) in the second storage (M2), and the synchronisation device (4) is preferably configured to automatically synchronise the further values stored in the storages (M1, M2, M3, M4) of the functional modules (CPU1, CPU2, CPU3, CPU4) with each other and/or to automatically restore said values.

8. Method for counting the operating hours of a medical device, preferably a dialysis device for performing a haemodialysis and/or haemofiltration and/or haemodiafiltration, which comprises a first functional module (CPU1) and a second functional module (CPU2), wherein in the first functional module (CPU1) the operating hours are determined with an operating hours counter (2) and the determined operating hours value is stored in a storage (M1) of the first functional module (CPU1),
**characterised in that**
the operating hours value determined by the operating hours counter (2) of the first functional module (CPU1) is stored in a second storage (M2) comprised by the second functional module (CPU2), wherein the first functional module (CPU1) is a main control module and the second functional module (CPU2) is a monitoring system.

9. Method according to claim 8, **characterised in that** the operating hours value determined by the operating hours counter (2) of the first functional module (CPU1) is stored in further storages (M2, M3, M4) of further functional modules (CPU2, CPU3, CPU4).

10. Method according to claim 8 or 9, **characterised in that** automatic synchronisation and/or restoring of the operating hours values is performed, when there is a deviation of the operating hours values stored in the storages (M1, M2, M3, M4).

11. Method according to claim 10, **characterised in that** the automatic synchronisation and/or restoring of the operating hours values is performed after a user input and/or after maintenance and/or the switching-on of the medical device (1) and/or according to schedule.

12. Method according to claim 10 or 11, **characterised in that** the automatic synchronisation and/or restoring of the operating hours values is performed when there is a predefined minimal deviation of the operating hours values stored in the storages (M1, M2, M3, M4).

13. Method according to any one of claims 10 to 12, **characterised in that** the operating hours value which is most frequently present in the storages (M1, M2, M3, M4) is used as a basis for the synchronisation and/or restoring of the operating hours values.

14. Method according to any one of claims 8 to 13, **characterised in that** further values are stored in the first storage (M1) of the first functional module (CPU1), preferably configuration values and/or setup values and/or calibration values, and the further values stored in the storage (M1) of the first functional module (CPU1) are stored in the second functional module (CPU2) in the second storage (M2), and preferably the further values stored in the storages (M1, M2, M3, M4) of the functional modules (CPU1, CPU2, CPU3, CPU4) are automatically synchronised with each other and/or are automatically restored.

## Revendications

1. Appareil médical (1), de préférence un dispositif de dialyse pour la réalisation d'une hémodialyse et/ou d'une hémofiltration et/ou d'une hémodiafiltration, comprenant un premier module fonctionnel (CPU1) et un deuxième module fonctionnel (CPU2), dans lequel est prévu un compteur horaire (2) dans le premier module fonctionnel (CPU1) pour compter les heures de fonctionnement, et le premier module fonctionnel (CPU1) est conçu pour enregistrer une valeur des heures de fonctionnement déterminée par le compteur horaire (2) dans une première mémoire (M1) du premier module fonctionnel (CPU1), dans lequel une deuxième mémoire (M2) est prévue dans le deuxième module fonctionnel (CPU2),
**caractérisé en ce que**
le deuxième module fonctionnel (CPU2) est conçu pour enregistrer la valeur des heures de fonctionnement déterminée par le compteur horaire (2) du premier module fonctionnel (CPU1) dans la deuxième mémoire (M2), dans lequel le premier module fonctionnel (CPU1) est un module de commande principal et le deuxième module fonctionnel (CPU2) est un système de monitoring, et le premier module fonctionnel (CPU1) et le deuxième module fonctionnel (CPU2) présentent respectivement leur propre microprocesseur.

2. Appareil médical (1) selon la revendication 1, **caractérisé en ce que** le premier module fonctionnel (CPU1) est un système d'exploitation et/ou le deuxième module fonctionnel (CPU2) est un système de protection.

3. Appareil médical (1) selon la revendication 1 ou 2, **caractérisé en ce qu' au** moins deux autres modules fonctionnels (CPU2, CPU3, CPU4) sont prévus en plus du premier module fonctionnel (CPU1), qui présentent respectivement leur propre mémoire (M2, M3, M4), dans lequel les autres modules fonctionnels (CPU2, CPU3, CPU4) sont conçus respectivement pour enregistrer la valeur des heures de fonctionnement déterminée par le compteur horaire (2) du premier module fonctionnel (CPU1) dans leur mémoire (M2, M3, M4) et dans lequel de préférence chaque module fonctionnel (CPU1, CPU2, CPU3, CPU4) présente son propre microprocesseur.

4. Appareil médical (1) selon l'une quelconque de revendications précédentes, **caractérisé en ce qu'** une interface de communication (3) est prévue entre le premier module fonctionnel (CPU1) et le deuxième module fonctionnel (CPU2), qui est conçue pour transmettre la valeur des heures de fonctionnement déterminée par le compteur horaire (2) du premier module fonctionnel (CPU1) à la deuxième mémoire (M2), de préférence à chaque mémoire (M2, M3, M4) des autres modules fonctionnels (CPU2, CPU3, CPU4).

5. Appareil médical (1) selon l'une quelconque des revendications précédentes, **caractérisé en ce qu'** un dispositif de synchronisation (4) est prévu, qui est conçu pour synchroniser automatiquement les unes avec les autres les valeurs des heures de fonctionnement enregistrées dans les mémoires (M1, M2, M3, M4) des modules fonctionnels (CPU1, CPU2, CPU3, CPU4) et/ou pour récupérer automatiquement les valeurs des heures de fonctionnement.

6. Appareil médical (1) selon l'une quelconque des revendications précédentes, **caractérisé en ce que** la mémoire (M1) prévue dans le premier module fonctionnel (CPU1) et/ou la mémoire (M2, M3, M4) prévue dans le deuxième module fonctionnel (CPU2) et/ou dans tous les modules fonctionnels (CPU2, CPU3, CPU4) est une mémoire non volatile.

7. Appareil médical (1) selon l'une quelconque des revendications précédentes, **caractérisé en ce que** la première mémoire (M1) du premier module fonctionnel (CPU1) est conçue pour enregistrer d'autres valeurs, de préférence des valeurs de configuration et/ou des valeurs de paramétrage et/ou des valeurs d'étalonnage, dans lequel le deuxième module fonctionnel (CPU2) est conçu pour enregistrer les autres valeurs enregistrées dans la mémoire (M1) du premier module fonctionnel (CPU1) dans la deuxième mémoire (M2) et le dispositif de synchronisation (4) est conçu de préférence pour synchroniser automatiquement les unes avec les autres les autres valeurs enregistrées dans les mémoires (M1, M2, M3, M4) des modules fonctionnels (CPU1, CPU2, CPU3, CPU4) et/ou pour les récupérer automatiquement.

8. Procédé pour compter les heures de fonctionnement d'un appareil médical, de préférence un dispositif de dialyse pour la réalisation d'une hémodialyse et/ou d'une hémofiltration et/ou d'une hémodiafiltration, l'appareil comprenant un premier module fonctionnel (CPU1) et un deuxième module fonctionnel (CPU2), dans lequel les heures de fonctionnement sont déterminées dans le premier module fonctionnel (CPU1) avec un compteur horaire (2) et la valeur des heures de fonctionnement déterminée est enregistrée dans une mémoire (M1) du premier module fonctionnel (CPU1),

**caractérisé en ce que** la valeur des heures de fonctionnement déterminée par le compteur horaire (2) du premier module fonctionnel (CPU1) est enregistrée dans une deuxième mémoire (M2) comprise dans le deuxième module fonctionnel (CPU2), dans lequel le premier module fonctionnel (CPU1) est un module de commande principal et le deuxième module fonctionnel (CPU2) est un système de monitoring.

9. Procédé selon la revendication 8, **caractérisé en ce que** la valeur des heures de fonctionnement déterminée par le compteur des heures de fonctionnement (2) du premier module fonctionnel (CPU1) est enregistrée dans d'autres mémoires (M2, M3, M4) d'autres modules fonctionnels (CPU2, CPU3, CPU4).

10. Procédé selon la revendication 8 ou 9, **caractérisé en ce que,** lors d'un écart entre les valeurs des heures de fonctionnement enregistrées dans les mémoires (M1, M2, M3, M4), une synchronisation et/ou une récupération automatique(s) des valeurs des heures de fonctionnement est(sont) réalisée(s).

11. Procédé selon la revendication 10, **caractérisé en ce que** la synchronisation et/ou la récupération automatique(s) des valeurs des heures de fonctionnement est(sont) réalisée(s) après une entrée utilisateur et/ou après une maintenance et/ou après l'activation de l'appareil médical (1) et/ou à tour de rôle.

12. Procédé selon la revendication 10 ou 11, **caractérisé en ce que** la synchronisation et/ou la récupération automatique(s) des valeurs des heures de fonctionnement est(sont) réalisée(s) lors d'un écart minimum prédéfini entre les valeurs des heures de fonctionnement enregistrées dans les mémoires (M1, M2, M3, M4).

13. Procédé selon l'une quelconque des revendications 10 à 12, **caractérisé en ce que** la valeur des heures de fonctionnement présente le plus fréquemment dans les mémoires (M1, M2, M3, M4) est utilisée comme valeur de base pour la synchronisation et/ou la récupération des valeurs des heures de fonctionnement.

14. Procédé selon l'une quelconque des revendications 8 à 13, **caractérisé en ce que** d'autres valeurs sont enregistrées dans la première mémoire (M1) du premier module fonctionnel (CPU1), de préférence des valeurs de configuration et/ou des valeurs de paramétrage et/ou des valeurs d'étalonnage, et les autres valeurs enregistrées dans la mémoire (M1) du premier module fonctionnel (CPU1) sont enregistrées dans le deuxième module fonctionnel (CPU2) dans la deuxième mémoire (M2) et les autres valeurs

23    **EP 3 768 350 B1**    24

enregistrées dans les mémoires (M1, M2, M3, M4) des modules fonctionnels (CPU1, CPU2, CPU3, CPU4) sont de préférence synchronisées automatiquement les unes avec les autres et/ou récupérées automatiquement.

**13**

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6423268 B1
- DE 4131247 A1
- US 20130190674 A1
- EP 1698361 A1